Europäisches Patentamt

European Patent Office

Office européen des brevets

(19)

(11) Veröffentlichungsnummer: **0 184 710**
**A2**

(12) # EUROPÄISCHE PATENTANMELDUNG

(21) Anmeldenummer: **85114957.5**

(22) Anmeldetag: **26.11.85**

(51) Int. Cl.⁴: **C 07 K 17/14**, B 03 C 1/00,
G 01 N 33/544, G 01 N 33/553,
G 01 N 33/68

(30) Priorität: **08.12.84 DE 3444939**

(43) Veröffentlichungstag der Anmeldung: **18.06.86**
**Patentblatt 86/25**

(84) Benannte Vertragsstaaten: **AT BE CH DE FR GB IT LI NL
SE**

(71) Anmelder: **BAYER AG, Konzernverwaltung RP
Patentabteilung, D-5090 Leverkusen 1 Bayerwerk (DE)**

(72) Erfinder: **Müller-Ruchholtz, Wolfgang, Prof. Dr. Dr.,
Tränkenberg 8, D-2300 Molfsee (DE)**
Erfinder: **Kandzia, Jörg, Dipl.-Biol., Bruckmühlenweg 11,
D-7815 Kirchzarten-Bruckmühle (DE)**
Erfinder: **Haas, Wolfgang, Dipl.-Bio-Chem.,
Hallgrafenstrasse 17, D-8230 Bad Reichenhall (DE)**
Erfinder: **Leyhausen, Gabriele, Dr. rer. nat.,
Hasseldieksdammerweg 49, D-2300 Kiel (DE)**

(54) **Magnetische Microspheres.**

(57)  Die Erfindung betrifft magnetische Microspheres (MIMS),
ein Verfahren zu ihrer Herstellung sowie ihre Verwendung zur
Trennung und Isolierung biologischer Partikeln, insbesondere
natürlicher oder artifizieller Zellen.

Die mit dem erfindungsgemäßen Verfahren erhaltenen
magnetischen Microspheres sind durch Quervernetzung stabilisiert und tragen an der Oberfläche funktionelle Gruppen, die
die kovalente Bindung von biologisch aktiven Liganden erlauben.

EP 0 184 710 A2

BAYER AKTIENGESELLSCHAFT          5090 Leverkusen, Bayerwerk
Konzernverwaltung RP
Patentabteilung                   Ad/ABc


## Magnetische Microspheres


Die Erfindung betrifft magnetische Microspheres (MIMS),
ein Verfahren zu ihrer Herstellung sowie ihre Verwendung
zur Trennung und Isolierung biologischer Partikeln, insbesondere natürlicher oder artifizieller Zellen.


Eine wesentliche Zielsetzung zellbiologischer und biochemischer Forschung liegt in der Isolierung biologischer
Partikeln. Dies sind einerseits homogene Subpopulationen
bestimmter Zellen aus heterogenen Gemischen, aber auch
zelluläre Fragmente verschiedener Art. Wichtige Forderungen betreffen hohe Ausbeuten, höchstmögliche Zellschonung während des Trennprozesses, einfacher und schneller
Prozeßablauf und niedrige Kosten. Die zur Zeit verfügbaren Methoden bleiben bezüglich dieser Anforderungen unbefriedigend, insbesondere bei Gemischen mit hohen Partikelzahlen, z.B. $10^6$-$10^{12}$ Zellen.


Zentrifugationsverfahren (s. Hutchins, D. and C.M. Steel,
in: Peters, H. Editor: Separation of cells and subcellular elements. 1979) trennen auf der Basis des physikalischen Parameters Dichte bzw. Volumen und lassen demnach die Spezifität vermissen.


Le A 23 431 -Ausland

Diesbezüglich wurde ein Fortschritt erreicht durch die Entwicklung von Festphasen- Affinitätstechniken auf der Grundlage serologischer Reaktionen. Durch die Fixierung von Antikörpern an Plastikoberflächen (siehe z.B. Panningverfahren: Wysocki, L.J. and V.L. Sato, 1978, Proc. Natl. Acad. Sci. 75: 2844; Basch, R.S. et al. 1983, J. Immunol. Methods 56:269), Glas oder Gelmaterialien (S. Basch, R.S. et.al., ebd.), die zu Säulen gepackt werden können, erlauben diese Methoden, Zellen oder zelluläres Material durch Unterscheidung von molekularen Oberflächenstrukturen, z.B. Antigenen, spezifisch zu trennen. Nachteile dieser Verfahren sind unspezifische Bindungsreaktionen mit Materialoberflächen, das Verstopfen von Säulen durch aggregierende Partikeln bzw. Zellen, Beeinträchtigung empfindlicher Zellen durch Scherkräfte, lange Prozeßzeiten und ganz besonders mangelnde Kapazität, große Mengen zu bewältigen.

Unspezifische Haftung, ungenügende Zellschonung und Scherkräfte können in Flüssigphasen-Verfahren, in denen der eigentliche Trennvorgang aus verdünnter Suspension erfolgt, fast gänzlich ausgeschaltet werden. Hier sind zu nennen: Zellimmunelektrophorese (H.H.P. Cohly et al., 1984, International Meeting Cell Electrophoresis, Rostock, GDR), FACS (Loken M.R. and A.M.Stall, 1982, J. Immunol. Methods 50:R85) und magnetische Zelltrenntechniken auf der Basis Antikörper-konjugierter magnetischer Microspheres. Gemeinsam ist allen Techniken die Kombination der serologischen Spezifität von Antikörpern mit einer steuerbaren physikalischen Kraft: elektrisches Feld, optische Erkennung oder Magnetfeld.

Le A 23 431

Obgleich z.B. der FACS gegenwärtig auf analytischem Gebiet - insbesondere durch den multifaktoriellen Charakter - den Stand der Technik repräsentiert, verbleiben für die beiden erstgenannten Techniken gewichtige Nachteile. Für den präparativen Einsatz besteht nur begrenzte Kapazität zur Bewältigung großer Zellmengen in angemessener Zeit.

Demgegenüber bietet der Einsatz magnetischer Microspheres bemerkenswerte Vorteile, da magnetische Separationsverfahren mit Microspheres billig und einfach im Prozeßverlauf sind und insbesondere auf präparativem Gebiet nicht nur zur Bewältigung großer Zellmengen ($10^6 - 10^{12}$ Zellen), sondern auch zur schnellen Isolierung anderer zellulärer Partikeln wirtschaftlich angemessen eingesetzt werden können.

In den vergangengen Jahren wurde aus diesen Gründen eine Vielzahl verschiedener polymerer Microspheres entwickelt (Methacrylat, Molday et al., 1977, Nature 268:437; Acrylat, Rembaum et al., 1978, Chem. Tech March: 182; Polyglutaraldehyd, Rembaum et. al., 1978, J. Immunol. Methods, 24:239; Acrolein, Margel et. al., 1982, J. Cell. Sci. 56:157; Dextran, Molday and McKenzie, 1982, J. Immunol. Methods 52:353).

Zolle et al. (1970, Int. J. Appl. Radiat. 21:155) stellten erstmals Microspheres aus Albumin durch ein Koagulationsverfahren her. Senyei et al. (1978, J. Appl. Phys. 49:3578) entwickelten magnetische Albumin-Microspheres durch die Inkorporation von magnetischem Eisenoxyd in die Partikel und nuzten diese zum Transport von Arzneimitteln ("drug carrier"). Dieselbe Arbeitsgruppe beschrieb

Le A 23 431

die Herstellung von magnetischen Microspheres aus Albumin, in die an Stelle des Arzneimittels Protein A als Ligand für Antikörper in die Matrix einkoaguliert wurde (Widder et al., 1979, Clin. Immunol. Immunopath., 14:395). Die Vorteile dieser Microspheres gegenüber den o.a. liegen in der simplen und unaufwendigen Herstellung, der einfachen Ligandenkopplung, der guten Suspendierbarkeit auch in proteinhaltigen Lösungen und der nur minimalen unspezifischen Bindung.

Eigene Untersuchungen deckten aber die Möglichkeit des Verlustes des nicht kovalent gebundenen, nur einkoagulierten, Liganden Protein A durch Matrixquellung auf. Weiterhin waren durch Verwendung nur dieses einen Liganden die Kopplungsmöglichkeiten auch für andere, für Trennungsabläufe geeignete Moleküle limitiert (Kandzia et al., 1984, J. Immunol. Methods, im Druck). Daher wurde nach einem Weg gesucht, das einfache Herstellungsverfahren magnetischer Microspheres aus Albumin oder auch anderen geeigneten Matrixproteinen so zu entwickeln, daß sowohl maximale Matrixstabilisierung als auch die kovalente Kopplung von Molekülen an die Microsphere-Oberfläche möglich wird.

Gegenstand der Erfindung sind magnetische Microspheres, im folgenden auch MIMS genannt, bestehend aus einer magnetischen Komponente, inkorporiert in ein koaguliertes Matrixprotein, dessen Oberfläche durch funktionelle Gruppen zur Bindung biologischer Partikeln aktiviert ist.

Le A 23 431

Die erfindungsgemäßen Magnetischen Microspheres bestehen aus einem geeigneten, stabilisierten Matrixprotein, z.B. Albumin, Ovalbumin, Casein, allgemein natürlichen globulären und anderen Proteinen, wie auch synthetischen Polypeptiden, z.B. Poly-L-Lysinen, u.a., in deren Matrix Elemente oder Verbindungen mit ferromagnetischen Eigenschaften, z.B. Fe, Ni, Co bzw. Verbindungen oder Legierungen dieser Elemente, die die gleichen Eigenschaften besitzen, oder ganz allgemein magnetisierbare Komponenten dieser Art, insbesondere "Ferrofluide", eingeschlossen sind und deren Oberfläche durch funktionelle Gruppen, insbesondere Aldehydgruppen, aktiviert ist. An diese Gruppen können unter geeigneten Bedingungen Proteine aller Art, insbesondere biologisch direkt aktive oder auch andere Aminogruppenhaltige und als Liganden dienende Moleküle, z.B. Diaminoheptan, Diaminoethyl, Diaminopropyl etc., Enzyme, Protein A, Avidin, Aminozucker oder Aminofette kovalent gebunden werden.

Das Matrixprotein der magnetischen Microspheres wird durch geeignete di- oder polyfunktionelle Vernetzungsmittel quervernetzt, wobei auf der Oberfläche des Matrixproteins freie funktionelle Gruppen des Vernetzungsmittels vorliegen.

Geeignete Vernetzungsmittel sind Aldehyde, insbesondere Glutaraldehyd, sowie Verbindungen aus der Gruppe bestehend aus Dimethyladipimidat·2HCl, Dimethylpimelimidat·2HCl, Dimethylsuberimidat·2HCl, Dimethyl-3,3'-dithiobis-propionimidat·2HCl, 2-Iminothiolan·HCl, Disuccinimidylsuberat, Bis/2-(succinimidooxycarbonyl-oxy)ethyl7sulfon, Disuccinimidyltartrat, Dithiobis-(succinimidylpropionat), Ethylenglycol bis(succinimidyl succinat), N-5-azido-2-nitrobenzoyloxy-succinimid,

Le A 23 431

p-Azidophenacylbromid, p-Azidophenylglyoxal, 4-Fluoro-3-nitrophenylazid, N-Hydroxysuccinimidyl-4-azido-benzoat, N-Hydroxysuccinimidyl-4-azidosalicylsäure, m-Maleinimidobenzoyl N-Hydroxysuccinimidester, Methyl-4-Azidobenzoimidat, p-Nitrophenyl 2-Diazo-3,3,3-trifluoropropionat, N-Succinimidyl-6-(4'-azido-2'-nitrophenylamino)-Hexanoat, Succinimidyl 4-(N-maleimido-methyl)cyclohexan-1-carboxylat, Succinimidyl-4-(p-maleimido-phenyl)butyrat, N-(4-azidophenylthio)phthalimid, Ethyl-4-azidophenyl 1,4-dithiobutyrimidat. HCl, N-Succinimidyl(4-azidophenyl-dithio)propionat, 1,5-Difluoro-2,4-dinitrobenzol, N-Succinimidyl-3-(2-pyridyldithio)propionat, 4,4'-Dithiobisphenylazid und Erythritolbiscarbonat.

Die Erfindung betrifft bevorzugt Aldehyd-aktivierte MIMS, bei denen die Aldehydfunktion lediglich als Kopplungsmolekül bzw. Mediator zur Bindung anderer Kopplungsmoleküle unter geeigneten Bedingungen dient, z.B. CNBR, Diazoverbindungen, Thiol-Gruppen oder Abstandshaltermoleküle (Spacermoleküle), die es erlauben, andere aktivierte Gruppen, wie z.B. Carbodiimide, auf die Oberfläche der MIMS zu bringen.

Bevorzugt sind ferner MIMS, deren chemische Struktur durch das jeweils verwendete Matrixprotein, das verwen-dete Vernetzungsmittel, die eingeschlossene magnetisier-bare Komponente und die funktionelle Gruppe an der Oberfläche gegeben ist.

Bevorzugt sind auch MIMS, die in Wasser, wäßrigen Puffern wie 0,1 M Tris, 0,1 M Glycin, 0,1 M Acetate, 0,1 M Citrat, 0,1 M Phosphat, insbesondere auch in 6 M Guanidin-HCl, 4 M Harnstoff und 3 M Thiocyanat, wie auch in alkoholischen

Le A 23 431

Lösungsmitteln, Aceton, aber auch in Gegenwart von Triton(R) oder Tween(R), stabil sind. Sie sind dagegen nicht stabil in Gegenwart von Proteasen.

Die Erfindung betrifft auch magnetische Microspheres, die in den o.a. Puffern über einen pH-Bereich von 2,5-9 stabil sind und in den o.a. Puffern nicht quellen.

Weiter sind bevorzugt solche MIMS, an deren funktionelle Aldehydgruppen Aminogruppen-tragende Moleküle kovalent gebunden werden, z.B. und insbesondere Protein A, Avidin, Lectine, Immunglobuline einschl. Antiglobuline, und Enzyme, jedoch nicht Proteasen. Die Erfindung schließt ein, daß im Anschluß an die kovalente Reaktion in Gegenwart von 0,1 M $NaHCO_3$ (+ 0,5 M NaCl bei pH 8-9) die entstandenen Schiffschen Basen zwischen Aldehyd und Aminogruppe des gekoppelten Moleküls mit, z.B., Natriumborhydrid zum sek. Amin reduziert und die verbliebenen freien Aldehydgruppen zu OH-Gruppen reduziert werden.

Damit erhalten die MIMS hydrophilen Charakter und sind insbesondere in wäßrigen Lösungsmitteln gut zu suspendieren.

Die erfindungsgemäßen MIMS können im aktivierten Zustand trocken und wasserfrei bei Raumtemperatur gelagert werden. Nach Bindung der Proteinmoleküle ist eine Lagerung bei 3-6°C in lyophilisiertem Zustand oder in Puffer in Gegenwart von 0,02-0,1 % $NaN_3$ empfehlenswert.

Die erfindungsgemäßen MIMS sind biologisch vollständig abbaubar und gegenüber lebenden Zellen nicht toxisch.

Le A 23 431

Weiterer Gegenstand der Erfindung ist ein Verfahren zur Herstellung magnetischer Microspheres (MIMS), bestehend aus einer magnetischen Komponente, inkorporiert in ein koaguliertes Matrixprotein, dessen Oberfläche durch funktionelle Gruppen zur Binding biologischer Partikel aktiviert ist, das dadurch gekennzeichnet ist, daß man

a) eine magnetische Komponente mit einem Matrixprotein mischt und daraus Koazervate bildet,

b) die Koazervate koaguliert und

c) mit einem geeigneten bi- oder polyfunktionellen Vernetzungsmittel vernetzt.

Bevorzugt werden die MIMS in einfachen methodischen Schritten in der jeweils gewünschten Größe (Bereiche von 0,1-10 µm) sehr homogen (Homogenitätsgrad 70-80 %) monodispers hergestellt. Das Verfahren erlaubt es, durch die entscheidende unmittelbare Kombination eines physikalischen mit einem chemischen Methodenschritt die Stabilisierung und Aktivierung der MIMS überraschenderweise gleichzeitig zu erzielen.

Die Erfindung betrifft bevorzugt auch die Herstellung von Aldehyd-aktivierten MIMS, bei denen anstelle des Pflanzenöls (wie weiter unten gezeigt) andere geeignete, mit Wasser nicht mischbare Lösungsmittel verwendet werden.

Le A 23 431

Die Herstellung von Aldehyd-aktivierten MIMS kann durch Emulsion unter Ultraschall, aber auch unter Verwendung von Homogenisatoren bzw. durch Sprühtechniken unter Verwendung von Preßluft mittels geeigneter Düsen erfolgen.

Zur Herstellung Aldehyd-aktivierter MIMS, bei denen das Emulgat nicht in heißes Öl eingeströmt wird, können auch Vernebelungstechniken unter Verwendung von Preßluft bzw. $N_2$ unter Druck über geeignete Düsen zum Einsprühen auf bzw. in heißes Öl verwendet werden.

Die Herstellung Aldehyd-aktivierter MIMS, bei denen die Emulsion, wie oben beschrieben, in einer geeigneten Kammer vernebelt wird, wobei die MIMS in der Heißluft der Kammer kondensieren und koagulieren, ist ebenfalls möglich.

Die Koagulation des Matrixproteins erfolgt bei 50°C bis 180°C und bevorzugt bei 100°C bis 150°C.

Zur Reinigung der MIMS von Öl kann Aceton verwendet werden.

Bei der Herstellung Aldehyd-aktivierter MIMS können bei der Aldehydaktivierung anstelle von Ethanol abs. zweckmäßigerweise auch andere geeignete, mit Wasser mischbare Lösungsmittel, z.B. Aceton, verwendet werden.

Le A 23 431

Allgemeine Arbeitsvorschrift für die Herstellung der
erfindungsgemäßen magnetischen Microspheres (MIMS)

---

Dieses Beispiel betrifft eine allgemeine Arbeitsvorschrift
für die Herstellung von MIMS mittels eines "Emulsions-
Koagulations-Vernetzungs-Verfahrens", an dessen Ende der
Aldehyd-aktivierte MIMS steht. Matrixprotein und magnetisierbare Komponente werden im Gewichtsverhältnis 10:1 bis 1:10,
üblicherweise im Gewichtsverhältnis 5:1 bis 1:5 und bevorzugt im Gewichtsverhältnis 1:3, bezogen auf 100 % Gesamttrockensubstanz, in einer sehr geringen Menge aqua bidest
(1-2000 µl, üblicherweise 100 - 500 µl) gelöst bzw. suspendiert und durch hochtouriges Rühren in einem definierten
Volumenüberschuß einer mit Wasser nicht mischbaren Flüssigkeit
homogenisiert (wobei sich die Verwendung von Pflanzenölen als zweckmäßig erwiesen hat) und zwar bei 10 - 40°C,
besonders bei 20 - 25°C, über 1 - 30 Minuten, üblicherweise
10 Minuten. Der Volumenüberschuß an Öl umfaßt den Bereich 10:1 - 1000:1 (v/v). Der Zusatz eines geeigneten
Emulgators, z.B. Span 80 ®(HLB 4,3 < 5), ist allgemein
als zweckmäßig anzusehen. Dieses Homogenat wird zu einer
Emulsion bereitet. Emulsionsverfahren, wie z.B. Ultrabeschallung definierter Stärke von 10 - 500 W, üblicherweise bei 100 - 150 W, und definierter Zeitdauer von
1 - 10 Minuten, gepulst wie auch stufenlos, haben sich
hierbei als besonders geeignet erwiesen. Dabei stellt
sich der Zustand der Emulsion so ein, daß sich sehr
kleine Wasserkoazervate annähernd gleicher Größe bilden,
in welchen Matrixsubstanz und magnetisierbare Komponente
im o.a. Mengenverhältnis enthalten sind. Um einer Entmischung der Emulsion vorzubeugen und deshalb dieselbe
zu stabilisieren, wird zweckmäßigerweise das Emulgat auf
0 - 10°C, besonders vorteilhaft auf 0 - 4°C abgekühlt.
Dieses Emulgat wird mittels eines speziellen Einspritz-

Le A 23 431

systems in 100-300 ml stark erhitztes (100-150°C) Öl, z.B. Pflanzenöl, eingeströmt. Dabei koaguliert das Matrixprotein aus den Koazervaten (wobei das Lösungsmittel Wasser verdampft) unter Einschluß der magnetisierbaren Komponente in solcher Weise, daß sich entsprechende Microspheres bilden und über einen bestimmten Zeitraum, der üblicherweise 5 - 15 Minuten, zweckmäßig 10 Minuten umfaßt, in der Hitze vorstabilisieren. Diese MIMS werden durch mehrmaliges Waschen, wobei sich wasserfreier Diethylether als vorteilhaft erwiesen hat, vom Öl gereinigt, zur Trockene eingeengt (durch vollständige Verdunstung des Ethers bei Raumtemperatur) und gewogen. Diese MIMS werden in einem bestimmten Mengenverhältnis, 10-50 mg/20 ml Ethanol abs. (w/v), in Gegenwart eines geeigneten Lösungsvermittlers, z.B. Tween 80Ⓡ (HLB 15 - 10), resuspendiert. Zu solcher Suspension wird eine bestimmte Menge, 1 - 25 % (v/v), eines Dialdehyds, insbesondere Glutaraldehyd, gegeben und die Reaktion zwischen MIMS und Aldehyd über 12-24 Stunden durch mildes Rühren durchgeführt.

Bei dieser Reaktion erfolgt überraschenderweise durch Quervernetzung des koagulierten Matrixproteins sowohl die endgültige physikalische Stabilisierung der MIMS-Matrix wie auch, durch den Dialdehydcharakter des Vernetzungsmittels, die Exposition funktioneller Aldehydgruppen an die MIMS-Oberfläche. Die Reaktion wird durch mehrmaliges Waschen der MIMS mit Ethanol abs. und in abnehmender Alkoholreihe mit aqua bidest. an einem Magneten beendet.

Le A 23 431

Beispiel für die Herstellung der erfindungsgemäßen magnetischen Microspheres (MIMS)

---

7,6 mg Rinderserumalbumin als Matrixprotein werden in 250 µl aqua dest gelöst, zu 100 mg Ferrofluid[®]-Lösung, die 18 % Eisenoxid enthält (Ferrofluidics Corporation, New Hampshire) gegeben und gut gemischt. Dieses Gemisch wird unter hochtourigem Rühren (1200 U/min, 2 min) zu 25 ml Pflanzenöl zugegeben. Anschließend wird die entstandene Emulsion durch Rühren bei 2000 U/min für 5 min weiter homogenisiert. Das Homogenat wird auf 4°C gekühlt, in ein im Eisbad gekühltes Rosettengefäß (Volumen 20 - 40 ml) gegeben und durch Beschallung mit Ultraschall (2 x 2 min, 1 x 1 min, 22 micron (Soniprep 150, 3/8 "Titansonde")) zu einer feinen Emulsion bereitet. Diese Emulsion wird mit einer 25 ml-Spritze aufgenommen und durch eine Kanüle (0,9 x 40 mm) in stark erhitztes, gerührtes Öl (120°C, 150 ml) innerhalb von 30 - 60 sec unter gleichmäßigem Druck gespritzt. Anschließend wird noch 10 min weitergerührt, wobei die gebildeten Microspheres in der Hitze vorstabilisiert werden. Mit dem so entstandenen Gemisch werden Zentrifugengläser (Vol.: 50 ml) zur Hälfte beschickt und auf 4°C gekühlt. Anschließend wird das gleiche Volumen wasserfreier Diäthyläther hinzugefügt, gemischt und abzentrifugiert (20 min, 2000xg). Anschließend wird der Überstand abdekantiert und die Microspheres (Pellet) in einem Glas vereinigt. Um Reste des Pflanzenöls zu entfernen, werden die Microspheres noch fünfmal mit Diäthyläther gewaschen, dann an der Luft getrocknet und gewogen. (Danach können die MIMS gelagert werden).

Stabilisierung und Aktivierung: 10 mg MIMS werden in 10 ml Aceton (Variation a) oder 10 ml Äthanol (Variation b) aufgenommen und 10 - 30 sec. mit 15 micron beschallt, um die

Le A 23 431

0184710

MIMS in dem Lösungsmittel zu suspendieren. Anschließend werden 8 ml des jeweiligen Lösungsmittels und 2 ml einer Glutaraldehydlösung zugesetzt,so daß die Endkonzentration an Glutaraldehyd im Reaktionsansatz 2,5 % beträgt.

Bei Zimmertemperatur wird die Reaktion entweder 30 Minuten in Aceton oder über Nacht (18 h) in Äthanol unter horizontaler Rollbewegung durchgeführt. Anschließend werden die Microspheres folgender Waschprozedur mit je 5 ml der folgenden Lösungen unterworfen:

    3 x Aceton
    2 x Aceton: 0,1% Tween 80 in aqua dest = 4:1
    2 x Aceton: 0,1% Tween 80 in aqua dest = 1:1
    2 x Aceton: 0,1% Tween 80 in aqua dest = 1:2
    2 x 0,1% Tween 80 in aqua dest
    3 x Phosphat-gepufferte Kochsalzlösung

Die MIMS sind bei diesem Waschvorgang an einem Magneten fixiert. Anstelle von Aceton tritt im Falle der Aktivierungsvariante b Äthanol. Danach sind die Microspheres ohne Stabilitätsverlust entweder in 0,1% Azid in Phosphat-gepufferter Kochsalzlösung (pH 7,2) oder in gefriergetrocknetem Zustand aufzubewahren.

Le A 23 431

**Anwendungsbeispiel für die magnetischen Microspheres**

Die erfindungsgemäßen MIMS wurden auf ihre Eignung als Trennreagenz für die magnetische Zellseparation in verschiedenen Trennungsexperimenten überprüft.

An Glutaraldehyd-aktivierte MIMS wurde zunächst Protein A als Ligand kovalent gebunden und anschließend ein monoclonaler anti-HLA- BW6-Antikörper über diesen Liganden an die MIMS gekoppelt. Diese MIMS wurden zur Auftrennung von physikalisch besonders homogenen Modell-Zellgemischen aus fraktionierten peripheren Blut-Lymphozyten von gesunden Blutspendern eingesetzt, die sich lediglich durch die HLA-Gewebetypen BW4 und BW6 unterschieden.

a)  Aufbereitung der Zellen

Nach Entnahme von 5 - 10 ml Blut und Eingabe in mit 1-3 Tropfen Liquemin o.ä. beschickte Glasröhrchen werden die Proben 1:1 mit PBS, pH 7,2 (handelsübliche Zusammensetzung) verdünnt. Von dieser Blut-Puffer-Mischung werden jeweils 4 ml über 3 ml Lymphozytentrennmedium (Dichte 1,077 g/l) geschichtet und 40 Minuten 600 x g bei Zimmertemperatur zentrifugiert. Anschließend werden die an der Trennschicht liegenden Zellen abgezogen, gesammelt, 3 x mit PBS (8000 mg/l NaCl, 600 mg/l KCl, 1440 mg/l $Na_2 HPO_4 \cdot H_2O$, 200 mg/l $KH_2PO_4$, pH 7,2-7,3) gewaschen und in Medium RPMI 1640 (Zusammensetzung, siehe Tabelle 1) inklusive 6 % BSA (Globulin- und Fettsäure-frei) aufgenommen. Nach der Zellzählung (Neubauer-Kammer) und der Vitalitätsbe-

Le A 23 431

stimmung (Trypanblau-Test) werden je 1 x 10$^6$ BW4$^+$-Lymphozyten und BW6$^+$-Lymphozyten von verschiedenen Blutspendern gemischt und mit 1 mg MIMS 30 Minuten bei Zimmertemperatur (22°C) unter konstantem Schütteln inkubiert. Danach wird der prozentuale Anteil der Zellen bestimmt, welche MIMS gebunden haben.

b)  Zelltrennung

Über eine Probenschlinge wird die gesamte Zellsuspension in eine Trennvorrichtung eingebracht. Nach Einschalten von 3 hintereinander liegenden Trennmagneten wird die Zellsuspension mittels einer Peristaltikpumpe in einem kontinuierlichen "Free-Flow" System mit einer Durchflußrate von 0,4 ml/min. an den Magneten vorbeigeführt.

Die Trennung findet bei Zimmertemperatur statt. Die Trenndauer liegt zwischen 10-20 Minuten. Es wird das doppelte Flüssigkeitsvolumen der Trenneinheit als Eluatfraktion gesammelt. Anschließend werden die Magnete abgeschaltet und wird die Magnetfraktion aus dem System durch pulsartiges Ausspülen mittels der Pumpe gewonnen.

c)  Repräsentatives Ergebnis einer solchen Stichproben-Untersuchung

c)1) Untersuchung der Vitalität nach der Aufbereitung der Zellen: Die Vitalität liegt üblicherweise zwischen 95 und 100 %.

Le A 23 431

c)2) Bindung der MIMS an die Lymphozyten:

Bei einem Gemisch von 1:1 bezüglich BW4$^+$ und BW6$^+$ Lymphozyten findet man nach 30-minütiger Inkubation, daß 45-52 % der Zellen MIMS gebunden haben und 48-55 % nicht.

c)3) Untersuchung der Fraktionen nach der Trennung im Magnetfeld:

Reinheit des Eluats nach nur einmaligem Durchlauf:

Unter den Zellen im Eluat findet man üblicherweise 0-4 % Zellen, die MIMS gebunden haben.

c)4) Reinheit der Magnetfraktion nach nur einmaligem Durchlauf:

Unter den Zellen in der Magnetfraktion findet man üblicherweise 5-15 % der Zellen, die keine MIMS gebunden haben.

c)5) Rückgewinnung der Zellen aus dem System:

Von den Zellen, die man eingesetzt hat (2 x 10$^6$ Zellen), findet man in der Regel 95-100 % wieder.

Damit liegt die Trennleistung des Gesamtsystems, die in der Hauptsache bedingt ist durch die biologische Spezifität des Antikörpermoleküls und die physikalische Auswirkung des Magnetfeldes, im Bereich der Eluatfraktion

Le A 23 431

zwischen 96-100 % und im Bereich der Magnetfraktion zwischen 85-95 % bei einem einmaligen Durchlauf des Zellgemisches durch das magnetische Feld. Ein zweiter Durchlauf ist möglich und für manche Fragestellungen prinzipiell wünschenwert. Daß es sich um eine sehr schonende Separationsmethode handelt, zeigt die kaum beeinträchtigte Vitalität der Zellen, die nach der Trennung noch zwischen 92-99 % liegt. Es ist offensichtlich, daß das Verfahren sowohl analytisch wie präparativ einsetzbar ist.

## Anwendungsperspektiven der magnetischen Microspheres

Die erfindungsgemäßen MIMS können zur Auftrennung biologischer Partikeln, insbesondere Zellen, aus heterogenen Gemischen, insbesondere natürlichen und/oder artifiziellen Zellgemischen (z.B. aus Gemischen von Tier bzw. Pflanzenzellen, transformiert und untransformiert, Pilzzellen, Bakterienzellen) angewendet werden, sofern die Partikeln über spezifische Oberflächenstrukturen unterscheidbar sind, z.B. serologisch mit Hilfe von Antikörpern oder biochemisch mit Hilfe von Lectinen, Enzymen oder anderen geeigneten Molekülen.

Die erfindungsgemäßen MIMS können ferner zur Isolierung von Zellfragmenten, insbesondere Rezeptor-tragenden Membranbestandteilen aus Zellhomogenaten, mit Hilfe von Antikörpern, Lectinen oder sonstigen gegen Zellkomponenten gerichteten Molekülen, angewendet werden.

Le A 23 431

Die Erfindung betrifft auch die Anwendung von MIMS zur schnellen und wirtschaftlichen Anreicherung von Kulturzellen, die bestimmte Moleküle produzieren, sowie die Isolierung dieser Moleküle aus Kulturflüssigkeiten.

Schließlich betrifft die Erfindung auch solche MIMS, die nach Bindung von Proteinen, Peptiden und kleineren Molekülen (Haptenen) als Träger (Adjuvantien) bei Immunisierungen angewendet werden können.

Le A 23 431

## Tabelle 1: Zusammensetzung von Medium RMPI1640

| Aminosäuren | mg/l | Vitamine | | Sonstige Substanzen | |
|---|---|---|---|---|---|
| L-Arginin | 200,0 | D-Biotin | 0,2 | D(+)-Glucose·$H_2O$ | 2200,0 |
| L-Asparagin·$H_2O$ | 56,82 | Ca-D(+)-Pantothenat | 0,25 | Glutathion | 1,0 |
| L-Asparaginsäure | 20,0 | Cholinchlorid | 3,0 | Phenolrot, Na | 5,3 |
| L-Cystin·2 HCl | 65,17 | Folsäure | 1,0 | | |
| L-Glutamin | 300,0 | meso-Inosit | 35,0 | Anorganische Salze | |
| L-Glutaminsäure | 20,0 | Nicotinsäureamid | 1,0 | | |
| Glycin | 10,0 | p-Aminobenzoesäure | 1,0 | Ca(NO$_3$)$_2$·4 $H_2O$ | 100,0 |
| L-Histidin | 15,0 | Pyridoxin ·HCl | 1,0 | KCl | 400,0 |
| L-Hydroxyprolin | 20,0 | Riboflavin | 0,2 | MgSO$_4$·7 $H_2O$ | 100,0 |
| L-Isoleucin | 50,0 | Thiamin·HCl | 1,0 | NaCl | 6000,0 |
| L-Leucin | 50,0 | Vitamin B12 | 0,005 | Na$_2$HPO$_4$·2 $H_2O$ | 1003,6 |
| L-Lysin·HCl | 40,0 | | | NaHCO$_3$ | 2000,0 |
| L-Methionin | 15,0 | | | | |
| L-Phenylalanin | 15,0 | | | | |
| L-Prolin | 20,0 | | | | |
| L-Serin | 30,0 | | pH 7,2 | | |
| L-Threonin | 20,0 | | | | |
| L-Tryptophan | 5,0 | | | | |
| L-Tyrosin | 20,0 | | | | |
| L-Valin | 20,0 | | | | |

## Patentansprüche

1. Magnetische Microspheres (MIMS), bestehend aus einer magnetischen Komponente, inkorporiert in ein koaguliertes Matrixprotein, dadurch gekennzeichnet, daß die Oberfläche des Matrixproteins durch funktionelle Gruppen zur Bindung biologischer Partikeln aktiviert ist.

2. Magnetische Microspheres nach Anspruch 1, dadurch gekennzeichnet, daß das Matrixprotein mit einem oder mehreren bi- oder mehr-funktionellen Vernetzer(n) vernetzt ist.

3. Magnetische Microspheres nach den Ansprüchen 1 und 2, dadurch gekennzeichnet, daß die Oberfläche des Matrixproteins durch Aldehydgruppen aktiviert ist.

4. Magnetische Microspheres nach Anspruch 3, dadurch gekennzeichnet, daß mittels der Aldehydfunktionen von der Aldehydfunktion verschiedene Kopplungsgruppen bzw. Kopplungsmoleküle an die Oberfläche des Matrixproteins gebunden sind.

5. Magnetische Microspheres nach den Ansprüchen 1 und 2, dadurch gekennzeichnet, daß das Matrixprotein mit Vernetzern aus der Gruppe bestehend aus Dimethyl Adipimidat·2HCl, Dimethylpimelimidat·2HCl, Dimethylsuberimidat·2HCl, Dimethyl 3,3'-dithio-bis-propionimidat·2HCl, 2-Iminothiolan HCl, Disuccinimidylsuberat, Bis/succinimidooxycarbonyl-oxy)ethyl/sulfon, Disuccinimidyltartrat,

Le A 23 431

Dithiobis(succinimidylpropionat), Ethylglycol-bis(succinimidylsuccinat), N-5-Azido-2-nitrobenzoyl-oxy-succinimid, p-Azidophenacylbromid, p-Azidophenylglyoxal, 4-Fluoro-3-nitrophenylazid, N-Hydroxysuccimidyl-4-azido-benzoat, N-Hydroxysuccini-midyl-4-azidosalicylsäure, m-Maleimidobenzoyl-N-hydroxysuccinimid-ester, Methyl-4-azidobenzoimidat ·HCl, p-Nitrophenyl-2-Diazo-3,3,3-trifluoropropio-nat, N-Succinimidyl-6-(4'-azido-2'-nitrophenylamido)-hexanoat, Succinimidyl-4-(N-maleimido-methyl)cyclohexan-1-carboxylat, Succinimidyl-4-(p-maleimido-phenyl)butyrat, N-(4-Azidophenylthio)phthalimid, Ethyl-4-azidophenyl-1,4-dithiobutyrimidat. HCl, N-Succinimidyl-(4-azidophenyl-dithio)propionat, 1,5-Difluoro-2,4-dinitrobenzol, 4,4'-Difluoro-3,3'-dinitrodiphenylsulfon, 4,4'-Diisothiocyano-2,2'-disulfonsäure, N-Succinimidyl-3-(2-Pyridyldithio)-propionat, 4,4'-Dithiobisphenylazid und Erythritolbiscarbonat vernetzt wird.

6. Magnetische Microspheres nach den Ansprüchen 1 bis 5, dadurch gekennzeichnet, daß das Matrixprotein ein globuläres Protein, insbesondere Albumin oder Ovalbumin ist.

7. Magnetische Microspheres nach den Ansprüchen 1 bis 6, dadurch gekennzeichnet, daß die magnetische Komponente aus Eisen und/oder Nickel und/oder Cobalt oder Verbindungen oder Legierungen dieser Elemente besteht.

Le A 23 431

8. Magnetische Microspheres nach den Ansprüchen 3 und 4, dadurch gekennzeichnet, daß das Vernetzungsmittel Glutaraldehyd ist und die funktionellen Gruppen an der Matrixproteinoberfläche aus diesem Vernetzer stammen.

9. Magnetische Microspheres (MIMS) nach den Ansprüchen 1 bis 8, dadurch gekennzeichnet, daß sie über die funktionellen Gruppen auf ihrer Matrixoberfläche an biologische Partikel gebunden sind.

10. Magnetische Microspheres nach Ansprüchen 1-9, dadurch gekennzeichnet, daß sie an natürliche oder artifizielle Zellen gebunden sind.

11. Magnetische Microspheres nach Anspruch 10, dadurch gekennzeichnet, daß sie an Zellfragmente gebunden sind.

12. Magnetische Microspheres nach Anspruch 9, dadurch gekennzeichnet, daß sie an Antikörper, Lectine oder sonstige gegen Zellkomponenten oder -fragmente gerichtete Strukturen gebunden sind.

13. Magnetische Microspheres nach Anspruch 9, dadurch gekennzeichnet, daß sie an Proteine, Peptide oder Haptene gebunden sind.

14. Verfahren zur Herstellung magnetischer Microspheres (MIMS), bestehend aus einer magnetischen Komponente,

Le A 23 431

inkorporiert in ein koaguliertes Matrixprotein,
dessen Oberfläche durch funktionelle Gruppen zur
Bindung biologischer Partikel aktiviert ist, dadurch gekennzeichnet, daß man

a)   eine magnetische Komponente mit einem Matrixprotein mischt und daraus Koazervate bildet,

b)   die Koazervate koaguliert und

c)   mit einem geeigneten bi- oder mehrfunktionellen
     Vernetzungsmittel vernetzt.

15.  Verwendung von magnetischen Microspheres (MIMS), bestehend aus einer magnetischen Komponente, inkorporiert in ein koaguliertes Matrixprotein, dessen
     Oberfläche durch funktionelle Gruppen zur Bindung
     biologischer Partikel aktiviert ist, zur Bindung
     und/oder Trennung biologischer Partikel.

Le A 23 431

0184710

for  Austria
für  Österreich
pour l'Autriche

Patentansprüche:

1. Verfahren zur Herstellung magnetischer Microspheres (MIMS), bestehend aus einer magnetischen Komponente, inkorporiert in ein koaguliertes Matrixprotein, dessen Oberfläche durch funktionelle Gruppen zur Bindung biologischer Partikel aktiviert ist, dadurch gekennzeichnet, daß man

a)   eine magnetische Komponente mit einem Matrixprotein mischt und daraus Koazervate bildet,

b)   die Koazervate koaguliert und

c)   mit einem geeigneten bi- oder mehrfunktionellen Vernetzungsmittel vernetzt.

2. Verfahren nach Anspruch 1, dadurch gekennzeichnet, daß man die Koazervate durch Emulgieren oder mittels Sprüh- bzw. Vernebelungstechniken herstellt.

3. Verfahren nach Anspruch 2, dadurch gekennzeichnet, daß eine Emulsion einer wäßrigen Lösung von Matrixprotein und einer magnetischen Komponente in einer mit Wasser nicht mischbaren Flüssigkeit hergestellt wird.

4. Verfahren nach den Ansprüchen 2 und 3, dadurch gekennzeichnet, daß zur Herstellung der Emulsion Ultraschall, Homogenisatoren, Hochgeschwindigkeitsmischer oder ähnliches eingesetzt werden.

5. Verfahren nach den Ansprüchen 1 bis 4, dadurch gekennzeichnet, daß die Koagulation durch Hitze erfolgt.

Le A 23 431-EAT

6. Verfahren nach den Ansprüchen 1 bis 5, dadurch gekennzeichnet, daß die Koagulation in einer heißen, mit Wasser nicht mischbaren Flüssigkeit erfolgt.

7. Verfahren nach den Ansprüchen 1 bis 6, dadurch gekennzeichnet, daß man die Koagulation in 50 $^\circ$C bis 180 $^\circ$C heißem Öl durchführt.

8. Verfahren nach den Ansprüchen 1 bis 7, dadurch gekennzeichnet, daß man die Vernetzung in einem geeigneten Lösungsmittel durchführt.

9. Verfahren nach den Ansprüchen 1 bis 8, dadurch gekennzeichnet, daß man als Vernetzungsmittel solche aus der Gruppe bestehend aus Dimethyladipimidat·2HCl, Dimethylpimelimidat·2HCl, Dimethylsuberimidat·2HCl, Dimethyl 3,3'-dithio-bis-propionimidat·2HCl, 2-Iminothiolan·HCl, Disuccinimidylsuberat, Bis/succinimido-oxycarbonyloxy)ethyl7sulfon, Disuccinimidyltartrat, Dithiobis(succinimidylpropionat), Ethylglycol-bis(succinimidylsuccinat), N-5-Azido-2-nitrobenzoyl-oxy-succinimid, p-Azidophenacylbromid, p-Azidophenylglyoxal, 4-Fluoro-3-nitrophenylazid, N-Hydroxysuccinimidyl-4-azido-benzoat, N-Hydroxysuccinimidyl-4-azidosalicylsäure, m-Maleimidobenzoyl-N-hydroxysuccinimidester, Methyl-4-azidobenzoimidat·HCl, p-Nitrophenyl-2-diazo-3,3,3-trifluoropropionat, N-Succinimidyl-6-(4'-azido-2'-nitrophenylamino)-Hexanoat, Succinimidyl-4-(N-maleimido-methyl)cyclohexan-1-carboxylat, Succinimidyl-4-(p-maleimido-

Le A 23 431

phenyl-butyrat, N-_4-Azidophenylthio)phthalimid,
Ethyl-4-azidophenyl-1,4-dithiobutyrimidat 'HCl,
N-Succinimidyl-(4-azidophenyl-dithio)propionat, 1,5-
Difluoro-2,4-dinitrobenzol, 4,4'-Diisothiocyano-2,2'-
disulfonsäure, N-Succinimidyl-3-(2-pyridyldithio)-
propionat, 4,4'-Dithiobisphenylazid und Erythritolbiscarbonat verwendet.

10. Verfahren nach den Ansprüchen 1 bis 9, dadurch gekennzeichnet, daß man Glutaraldehyd als Vernetzungsmittel
und Ethanol in Anwesenheit eines Lösungsvermittlers als
Lösungsmittel verwendet.

11. Verfahren zur Bindung und/oder Trennung von biologischen
Partikeln, dadurch gekennzeichnet, daß man magnetische
Microspheres (MIMS), bestehend aus einer magnetischen
Komponente, inkorporiert in ein koaguliertes Matrixprotein einsetzt, dessen Oberfläche durch funktionelle
Gruppen zur Bindung biologischer Partikel aktiviert ist.

12. Verfahren nach Anspruch 11 zur Trennung und Reinigung
von Zellen und Zellfragmenten in heterogenen Gemischen
natürlicher oder artifizieller Zellen.

13. Verfahren nach Anspruch 11 zur Isolierung von Zellen
aus Kulturmedien.

14. Verfahren nach Anspruch 11 zur Isolierung von Proteinen,
Peptiden und Haptenen.

Le A 23 431